Europäisches Patentamt

European Patent Office

Office européen des brevets

(11) Numéro de publication : **0 068 954
B1**

# (12) FASCICULE DE BREVET EUROPÉEN

(45) Date de publication du fascicule du brevet :
26.09.84

(21) Numéro de dépôt : **82401053.2**

(22) Date de dépôt : **10.06.82**

(51) Int. Cl.³ : **A 61 K 31/485** // (A61K31/485,
31/47)

(54) **Nouvelle composition pharmaceutique à activité antalgique.**

(30) Priorité : **12.06.81 FR 8111632**

(43) Date de publication de la demande :
**05.01.83 Bulletin 83/01**

(45) Mention de la délivrance du brevet :
**26.09.84 Bulletin 84/39**

(84) Etats contractants désignés :
**BE CH DE GB IT LI LU NL SE**

(56) Documents cités :
**FR-M- 7 606
UNLISTED DRUGS, vol. 22, no. 5, mai 1970, Chatham,
N.J., (USA); page 69d, "CHINOPYRIN"
UNLISTED DRUGS, vol. 31, no. 2, février 1979,
Chatham, N.J., (USA); page 24d, "KODISAL"
UNLISTED DRUGS, vol. 31, juin 1979, Chatham, N.J.,
(USA); page 90d, "PHENAPHEN 650"
DICTIONNAIRE VIDAL, 1974, OVP, Paris (FR); page
1038, "MIGRALGINE"
ROTE LISTE, 1971, Cantor, Aulendorf/Württ (DE);
page 144, "BARALGIN COMPOSITUM"
DICTIONNAIRE VIDAL, 1971, OVP, Paris (FR); page
19, "ADALGUR"**

(73) Titulaire : **PIERRE FABRE S.A.
125, rue de la Faisanderie
F-75116 Paris (FR)**

(72) Inventeur : **Dubois, Jacques
61, avenue de Rouqueourbe
F-81100 Castres (FR)**
Inventeur : **Fabre, Jacques
91 bis avenue de Lautrec
F-81100 Castres (FR)**

(74) Mandataire : **Schrimpf, Robert et al
Cabinet Regimbeau 26, Avenue Kiéber
F-75116 Paris (FR)**

**Description**

La présente invention a pour objet une nouvelle association antalgique composée de deux substances connues :
— la Glafénine ou α-monoglycéride de la 4(2′-carboxyphénylamino)-7-chloro-quinoléine,
— la Codéine ou méthylmorphine, alcaloïde de l'opium à noyau phénanthrénique.

Ces deux substances présentent normalement et isolément une activité antalgique non dénuée d'effets secondaires aux doses utilisées.

La Glafénine présente une activité :
— antalgique par action périphérique ou centrale selon les auteurs,
— anti-inflammatoire,
— antipyrétique faible.

La Glafénine a probablement une activité inhibitrice des prostaglandines. Les doses usuelles de Glafénine, pour que celle-ci présente une activité antalgique suffisante, sont de 200 à 400 mg par unité de prise (la dose journalière étant au maximum de 1 200 mg).

A ces doses, il a été signalé des accidents de type allergique et quelques cas d'insuffisance rénale.

La Codéine présente une activité :
— antalgique d'action centrale,
— antitussive.

La Codéine présente une activité antalgique à des doses allant de 10 à 60 mg par unité de prise. Des doses élevées de Codéine provoquent des effets secondaires sur le système digestif (constipation, nausées, vomissements), sur le système respiratoire (par diminution de l'excitabilité du centre respiratoire bulbaire). Des réactions allergiques peuvent se produire. De même, l'utilisation prolongée de fortes doses de Codéine produit une dépendance de type morphinique.

La Glafénine a été associée à un décontracturant (Thiocolchicoside) et à un anxiolytique (Meprobamate) dans une spécialité plus particulièrement destinée aux syndromes douloureux avec participation musculaire.

La Codéine a été assez fréquemment associée à d'autres antalgiques tels que l'aspirine ou le paracétamol.

L'association de Glafénine et de Codéine constitue une nouvelle association antalgique qui permet d'observer, à des doses convenablement choisies mais inférieures aux doses thérapeutiques usuelles :
— une potentialisation de l'effet antalgique,
— un élargissement du spectre d'activité,
— une diminution des effets secondaires.

La tolérance digestive de l'association est, par ailleurs, excellente.

La composition selon la présente invention est caractérisée en ce qu'elle comporte, à titre de principes actifs, au moins de la Glafénine et de la Codéine libres ou sous forme de sel et un support acceptable en pharmacie.

Le rapport en poids Codéine/Glafénine est de préférence compris entre 1/10 et 1/5 dans les compositions administrables par voie orale. Dans ce type de compositions, chaque unité de prise comprend de préférence de 100 à 150 mg de Glafénine base et de 10 à 30 mg de Codéine base.

Cette nouvelle association présente une activité antalgique intense, rapide, apparaissant dans les 20 à 30 minutes qui suivent la prise orale et qui se maintient pendant 4 à 6 heures. Le produit est éliminé en totalité au bout de 24 heures.

Cette nouvelle composition peut se présenter sous une forme quelconque administrable par voie orale, telle que comprimés, gélules, chronules à libération programmée ou même sous forme liquide.

Dans certains cas, l'administration par voie rectale sous forme de suppositoire pourra être envisagée, elle nécessite alors l'emploi de chlorhydrate de Glafénine et de phosphate de Codéine, de préférence aux doses suivantes :
— chlorhydrate de Glafénine : 200 à 300 mg
— phosphate de Codéine : 10 à 30 mg.

Les compositions selon la présente invention peuvent être formulées selon les techniques et avec des excipients connus dans ce domaine.

La présente invention concerne également un procédé de préparation des compositions antalgiques, caractérisé en ce qu'on mélange la glafénine, la codéine et au moins un excipient pharmaceutiquement acceptable, le mélange étant ensuite mis sous une forme galénique déterminée.

D'autres caractéristiques du procédé apparaîtront à la lecture de la présente description.

1. Tests pharmacologiques

1.1. Test de l'analgésie sur les tissus enflammés (Méthode de RANDALL & SELITTO)

Cette méthode est basée sur l'abaissement, par l'inflammation, du seuil de sensibilité à la douleur et sur son élévation par les analgésiques. Ce test présente l'avantage d'être applicable aussi bien aux analgésiques centraux que périphériques. Pour les analgésiques périphériques, l'effet analgésique ne s'exerce que sur la patte enflammée alors que les analgésiques centraux agissent sur la patte normale.

La douleur est provoquée par pression mécanique appliquée sur la face plantaire d'une patte.

— L'expérimentation porte sur les rats mâles pesant de 100 à 135 grammes (10 rats traités et 10 rats témoins).

— L'inflammation est provoquée par injection sous l'aponévrose plantaire de 0,1 ml de suspension de levure de bière à 20 %.

— Le seuil de douleur est mesuré sur la patte enflammée et sur la patte normale 1,2 et 4 heures après administration intragastrique du produit.

La composition selon la présente invention soumise à ce test est :

| | |
|---|---|
| Glafénine | 9 parties en poids, |
| Codéine | 1 partie en poids. |

Les résultats obtenus montrent un effet antalgique franc à la dose de 10 mg/kg sur la patte enflammée et un effet antalgique modéré à la dose de 20 mg/kg sur la patte normale. Cet effet persiste pendant les 4 heures de l'expérimentation.

### 1.2. Technique à la phénylbenzoquinone

Le syndrome douloureux provoqué par l'injection intrapéritonéale de 0,25 ml de solution de phénylbenzoquinone à 0,02 % chez la souris se caractérise par des mouvements d'étirements des pattes postérieures et de torsion dorso abdominale que l'on comptabilise pendant un laps de temps de 30 minutes à partir des 15 minutes qui suivent l'administration de la phénylbenzoquinone.

La substance antalgique est administrée par voie intragastrique à 10 souris par dose 30 minutes avant la phénylbenzoquinone. Un lot de 10 souris recevant du liquide physiologique est examiné parallèlement à titre de lot témoin.

On considère comme $AD_{50}$ la dose de produit qui chez 50 % des animaux réduit le nombre des mouvements à moins de la moitié du nombre moyen des mouvements des témoins.

La composition testée selon la présente invention est la suivante :

| | |
|---|---|
| Glafénine | 9 parties en poids, |
| Codéine | 1 partie en poids. |

Avec la composition testée on obtient une $AD_{50}$ de l'ordre de 250 mg/kg.

### 2. Etude clinique

Expérimentation contre placebo portant sur 50 personnes souffrant de douleurs diverses (aiguës et chroniques) :

— algies traumatiques,
— douleurs dentaires,
— douleurs menstruelles,
— algies rhumatismales (arthrose, arthrite, périarthrite, etc.),
— douleurs d'origine neurologique,
— douleurs viscérales,
— algies diverses ; céphalées d'éthilogies diverses, etc.

L'administration de l'association s'est faite à raison d'une prise orale d'un comprimé comportant :

| | |
|---|---|
| — Glafénine | 150 mg |
| — Codéine | 20 mg |

L'association amène une sédation des douleurs dans 83 % des cas.

### Revendications

1. Composition pharmaceutique, notamment à activité antalgique, caractérisée en ce qu'elle comporte, à titre de principes actifs, de la Glafénine et de la Codéine libres ou sous forme de sel et un support acceptable en pharmacie, dans ladite composition le rapport en poids Codéine/Glafénine est compris entre 1/10 et 1/5 dans les compositions administrables par voie orale et par voie rectale, chaque unité de la composition comprend de 200 à 300 mg de chlorhydrate de Glafénine et de 10 à 30 mg de phosphate de Codéine.

2. Composition pharmaceutique selon la revendication 1, caractérisée en ce que chaque unité de la composition administrable par voie orale comprend entre 100 et 150 mg de Glafénine et 10 et 30 mg de Codéine libres.

3. Composition pharmaceutique selon la revendication 1, caractérisée en ce que le Codéine est utilisée sous forme de phosphate de Codéine et que la Glafénine est utilisée sous forme de chlorhydrate de Glafénine.

4. Procédé de préparation d'une composition pharmaceutique, notamment à activité antalgique, caractérisé en ce que cette composition est obtenue par mélange de Glafénine et de Codéine libres ou sous forme de sel avec un support acceptable en pharmacie.

## Claims

1. Pharmaceutical composition, particularly with antalgic activity, characterised in that it comprises, by way of principal constituents free Glafenine and free Codeine or their salts and a pharmaceutically acceptable carrier, in the said composition the ratio by weight of Codeine/Glafenine is between 1/10 and 1/5 in compositions administrable orally and rectally, each unit of the composition has 200 to 300 mg of Glafenine chlorhydrate and 10 to 30 mg of Codeine phosphate.

2. Pharmaceutical composition according to claim 1, characterised in that each unit of orally administrable composition has between 100 and 150 mg of free Glafenine and 10 to 30 mg of free Codeine.

3. Pharmaceutical composition according to claim 1, characterised in that the Codeine is utilized as Codeine phosphate and that the Glafenine is utilized as Glafenine chlorhydrate.

4. Process for the preparation of a pharmaceutical composition, particularly with antalgic activity, characterised in that the composition is obtained by mixing free Glafenine and free Codeine or their salts with a pharmaceutically acceptable carrier.

## Ansprüche

1. Pharmazeutische Zusammensetzung, insbesondere mit schmerzstillender Wirksamkeit, dadurch gekennzeichnet, daß sie als Wirkstoff Glafenin und Codein in freier Form oder als Salz, sowie einen pharmazeutisch verträglichen Träger enthält, mit einem Gewichtsverhältnis von Codein/Glafenin von 1/10 bis 1/15 in oral verabreichbaren Zusammensetzungen und Anteilen von 200 bis 300 mg Glafenin-Hydrochlorid und 10 bis 30 mg Codeinphosphat je Einheit in rectal verabreichbaren Zusammensetzungen.

2. Pharmazeutische Zusammensetzung nach Anspruch 1, dadurch gekennzeichnet, daß jede Einheit der oral verabreichbaren Form zwischen 100 und 150 mg freies Glafenin und 10 und 30 mg freies Codein enthält.

3. Pharmazeutische Zusammensetzung nach Anspruch 1, dadurch gekennzeichnet, daß Codein in Phosphatform und Glafenin in Form von Glafeninhydrochlorid verwendet wird.

4. Verfahren zur Herstellung einer pharmazeutischen Zusammensetzung, insbesondere mit schmerzstillender Wirkung, dadurch gekennzeichnet, daß diese Zusammensetzung erhalten wird durch Mischung von Glafenin und Codein, in freier Form oder in Salzform, mit einem pharmazeutisch verträglichen Träger.